# EUROPEAN PATENT APPLICATION

(11) **EP 4 834 410 A1**
(43) Date of publication of application: **04.11.2026**
(21) Application number: 26175013.7
(22) Date of filing: 28.04.2026
(51) Int. Cl.: A61M 35/00, B43K 1/00, B43K 8/02

(54) **ANTI-DRIP DEVICE, AN APPLICATOR DEVICE COMPRISING THE ANTI-DRIP DEVICE, A METHOD OF ASSEMBLING THE APPLICATOR DEVICE AND AN INSERTION TOOL FOR USE WITH THE APPLICATOR DEVICE**

(30) Priority: 28.04.2025 DK PA202530253; 19.12.2025 DK PA202530866
(71) Applicant: Flemming Licht Holding ApS, 2800 Kongens Lyngby (DK)
(72) Inventor: Licht, Flemming, 2800 Kongens Lyngby (DK); Tjellesen, Frederik Walsted, 2450 København SV (DK)
(74) Representative: Holme Patent A/S

(57) **Abstract**

An anti-drip device (4) is configured for mounting to an applicator device (1) of the kind comprising a reservoir (2) holding a substance, and a nib (6) in fluid communication with the substance inside the reservoir (2). The anti-drip device (4) comprises an exterior tubular anti-drip part (16) configured for being situated around the reservoir (2), and an interior anti-drip part (23), which is arranged inside the exterior tubular anti-drip part (16), and having an interior bore (25) configured for receiving the nib (6). An applicator device (1) is provided with the anti-drip device.

## Description

The present invention relates to an anti-drip device configured for mounting to an applicator device of the kind comprising a reservoir for a substance and a nib in fluid communication with the substance inside the reservoir, in particular a liquid substance.

A capillary applicator pen works based on capillary action, where a liquid moves up or against the force of gravity within a narrow space, such as a thin tube. Liquid is thus fed from the reservoir to the nib by simple capillary action due to the internal capillary structure of the reservoir and of the nib. Capillary applicator pens and capillary action are well known to the skilled person and will not be described further here.

It is however a problem with some known applicator pens - in particular with capillary applicator pens - that if they are shaken too heavily, liquid may leaks from them. If the liquid has low or high pH, such as the low pH of formic acid, acetic acid, or trichloroacetic acid, or the high pH of NaOH or KOH, leakage may cause liquid application onto unintended spots causing burns, e.g. at healthy skin. Emphasis is made that the anti-drip device of the present invention can be used for other applicator devices that contains liquids than capillary applicator pens, - to thereby further ensure that any capillary flow of liquid that might happen during storage and shaking of the pen does not escape to the surroundings.

It is a main aspect of the present invention to provide an anti-drip device for an applicator device, in particular for a capillary applicator pen.

In a further aspect is provided an anti-drip device that is inexpensive and easy to implement in the applicator device.

The novel and unique features whereby these and other aspects are achieved according to the present invention consist in that the anti-drip device comprises
- an exterior tubular anti-drip part configured for being situated around the reservoir, and
- an interior anti-drip part, at least a part of which is arranged inside the exterior tubular anti-drip part, and having an interior bore configured for sealingly receiving the nib.

The interior anti-drip part is designed to seal around the nib, whereas the exterior tubular anti-drip part is designed to seal around a nib-mounting end of the reservoir when the nib passes through the interior bore and into the reservoir. Thus the anti-drip device is configured to be mounted on the nib-mounting end of the reservoir.

In some preferred embodiments the interior anti-drip part is tubular.

Within the scope of the present invention the term "anti-drip" refers to the property of preventing unintended outlet of a substance from a substance delivery system, e.g. leakage of liquid out of a capillary pen when a capillary pen is shaken or subjected to elevated temperatures. Using the "anti-drip" device is thus an extra precaution to avoid spillage of liquid.

Furthermore, within the scope of the present invention the concept of that "at least a part of the interior anti-drip part is arranged inside the exterior tubular anti-drip part" includes any part spanning the diameter of an exterior proximal part of the exterior tubular anti-drip part, optionally including any part spanning the diameter of an outmost proximal opening of an exterior proximal annular wall part of the exterior tubular anti-drip part.

The reservoir may typically be a plastic pipe filled with compressed cotton, or other porous absorbing material, and being open at both ends, so that a capillary action can take place unobstructed.

In the context of the present invention the terms "distal" and "proximal" are used in the axial context of the components of the applicator device. The term "distal" denotes an end farthest away from the tip of the nib, and the term "proximal" denotes an opposite end towards the tip of the nib.

Advantageously an exterior annular wall part of the exterior tubular anti-drip part may comprise an exterior distal annular wall part having a first interior diameter that may be selected to fit sealingly around the nib-mounting end of the reservoir, either due to the exterior annular wall part of the exterior tubular anti-drip part being elastic, or due to the first interior diameter being substantial the same as the exterior diameter of the nib-mounting end of the reservoir. The exterior distal annular wall part of the exterior tubular anti-drip part may have an exterior diameter that does not exceed the interior diameter of a holder for the reservoir, e.g. a tubular holder, to be able to pass up through the lumen of the holder towards the nib-mounting end of the holder, such as e.g. being equal to or slightly less than the interior diameter of the holder.

In one embodiment the anti-drip device resembles a partly inverted sleeve.

In some embodiments of the present invention at least a part of an interior annular wall part of the interior anti-drip part may have a second interior diameter that is smaller than the first interior diameter to delimit a gap between the interior annular wall part and the exterior annular wall part.

The interior bore may have a fourth interior diameter that is smaller than or equal to the second interior diameter, whereby the interior bore is configured to fit sealingly around the nib, either due to the interior annular wall part of the interior anti-drip part being elastic, at least to an extent that the nib is not squeezed or compressed when mounted, so that its capillary property is lost, or due to the second interior diameter being substantial the same as the exterior diameter of the reservoir-mounting end of the nib.

Said nib passes further into the proximal part of the lumen of the reservoir, and the interior anti-drip part provides a mouth for easy passage of the nib to enter the reservoir. To assist in guiding the nib into the interior anti-drip part, said interior anti-drip part may advantageously be funnel-shaped, with a conical entry part that extends into a tube part further up inside the exterior tubular anti-drip part.

In order for the nib to enter the nib-mounting end of the reservoir the nib may have a rigid penetration means at its reservoir-inserting end, and/or the reservoir may have rupture means at its nib-facing end, or the reservoir may have a premade opening that may or may not be temporarily closed with a lid means. However preferably the reservoir is in form of a tubular member, such as a pipe, that fits into the holder and allows simple insertion of a more rigid reservoir-inserting end of the nib. Such a tubular member can advantageously be a sponge or cotton more or less saturated with the liquid substance.

In an advantageous embodiment of the anti-drip device according to the present invention the exterior distal annular wall part of the exterior tubular anti-drip part may extend into an exterior proximal annular wall part of the exterior tubular anti-drip part, at least a part of said exterior proximal annular wall part has a third interior diameter that is smaller than or equal to the first interior diameter, and at least an interior distal annular wall part of the interior anti-drip part has an exterior diameter that is equal to or smaller than the second interior diameter.

Optionally the interior distal annular wall part of the interior anti-drip part can be longer than the exterior proximal annular wall part of the exterior tubular anti-drip part so provide a long firm securing seat for the nib.

The anti-drip device can be structured to be fittingly mounted around the nib-facing end of the reservoir.

The anti-drip device may mate to be seated into a nib-facing end of a holder for the reservoir, thus the anti-drip device can have a coupling end shaped complementarily to the interior shape of the nib-facing end of said holder, to thereby provide for firm abutment and seating of the anti-drip device at said nib-facing end of said holder.

The above-mentioned relations between diameters may in some embodiments provide an annular gap between the exterior proximal annular wall part of the exterior tubular anti-drip part and the interior annular wall part of the interior anti-drip part, to thereby allow at least some resiliency or flexibility between said wall parts, in particular if the anti-drip device is made at least partly of a resilient, flexible, and/or elastomeric polymeric material.

In alternative advantageous embodiments the interior anti-drip part can be a ring-shaped body manufactured integral with the exterior annular wall, preferably integral with the exterior proximal annular wall part, and delimiting the bore for receiving the nib. The ring-shaped body may serve to make the proximal end of the anti-drip device structural stable, so that it does not deform during its sliding insertion into the holder when mounted at the nib-facing end of the reservoir. Such embodiments of an anti-drip device of the present invention can be made of any kind of materials including, but limited to, polymer, metal, ceramics, composite, and glass. The ring-shaped body may be a more or less flat solid body or be tubular.

Injection molding and thermoforming may serve to manufacture anti-drip devices of various materials, including making a single anti-drip device of materials having different physical and chemical properties. Non-polymeric sub-parts can be embedded in polymeric parts, and the exterior annular wall can have different properties than the interior annular wall. A preferred material for manufacturing any of the embodiments of the present invention may be an acid and/or alkali resistant polypropylene or polyethylene, as these materials are inexpensive and easy to injection mold or thermoform. Materials like polytetrafluorethylen (PTFE), polyether ether ketone (PEEK), and polyethylenimine (PEI), and similar polymers possessing same chemical and physical properties are however not excluded.

It may be preferred that the exterior tubular anti-drip part and the interior anti-drip part are arranged so that their respective exterior bore and interior bore are coaxial, whereby both the reservoir and the nib can be mounted in a fast and simple manner.

To further ensure that the anti-drip device is situated in the correct end position for inserting the nib and supporting the nib during displacing it to its position of use in fluid communication with the substance, such as a liquid substance, inside the reservoir, the proximal end of the anti-drip device can advantageously be shaped complementary to an interior seat at a proximal end of said holder to thereby also improve sealing between nib and anti-drip device. The proximal end of the anti-drip device and the interior seat can be made complementary in any convenient manner. As an example, the proximal end of the anti-drip device can e.g. have alternating male and female protrusions that mate into alternating female and male protrusions of the interior seat at the proximal end of the holder. Such interlocking of the anti-drip device and the holder may also serve as a means that prevents dislocation of the anti-drip device once the applicator pen is manipulated, both during assembling and in actual use.

In some embodiments the applicator device of the present invention, such as a capillary pen, may have a child proofing means that needs to be released by pressing and rotating an interior cap that covers the nib.

To further assist in a smooth inserting of the anti-drip device when mounted to the nib-facing end of the reservoir, some embodiments of the exterior tubular anti-drip part may be provided with at least one axially extending groove extending along the exterior annular wall. The groove(s) advantageously serve(s) as a venting air gap between the holder and the reservoir.

Said axially extending groove(s) may in some embodiments slide on corresponding and complementarily shaped rib(s) protruding radially and with similar distance as the axially extending groove(s) from the interior surface of the circumferential wall of the holder. In such a configuration the anti-drip device can expediently be slidingly guided in a controlled manner into its end position at the nib-facing end of the holder, where it is also further prevented from rotating due to e.g. the proximal end of the anti-drip device being shaped complementary to the interior seat of the holder for the reservoir, or due to frictional and/or resilient engagement with the neighboring parts or sub-parts of the applicator device.

Emphasis is made that the rib(s) in the alternative can be on the anti-drip device and the groove(s) be in the circumferential wall of the holder.

Irrespective of whether the holder has groove(s) or rib(s), such may also be an aid for venting around the reservoir, by providing a gap that prevents creation of an undesirable positive pressure that unintentionally forces substance out through the nib or around the nib.

Any of the previous described embodiments of a anti-drip device according to the present invention may have, at a distal annular edge, a plurality of spaced apart flexible protruding fingers to further facilitate insertion into the holder, and so that the reservoir can be mounted tightly inside the anti-drip device to further prevent back-flow of substance, such as of a liquid substance like acid.

The invention also concerns an applicator device configured with the above-described anti-drip device mounted onto a reservoir holding a substance in form of a liquid, preferably a liquid selected for use in the treatment of dermatological disorders, including local treatment of warts and fungus.

An applicator device provided with or without the anti-drip device of the present invention may further comprise an interior cap for surrounding at least a free end of the nib, so that there is no spillage during storage and so that evaporation can be efficiently avoided.

The anti-drip device of the present invention may further comprise an exterior proximal cap configured to enclose said interior cap, which exterior proximal cap may then serve the dual function of also being configured and arranged as a manual actuator of a child proofing means when first pushed towards the holder, and then rotated.

An opposite distal cap may be adapted to end-close a distal end of the holder after the reservoir with the liquid substance has been inserted.

Said interior cap and said exterior proximal cap may be integral with each other, in which case both can be demounted in a single step, or be detachable from each other, whereby the exterior proximal cap can be demounted first, and then the interior cap can be demounted. Any residual capillary action may or may not store small residual amounts of liquid so that the interior cap may be just minimum wet inside. The dual arrangement of an interior cap surrounded by an exterior proximal cap provides an extra safety against any residual liquid substances that might be inside the interior cap, e.g. acidic liquids, unintentionally and uncontrolled escapes from the applicator device.

During storage, non-use and/or shaking of the applicator device the capillary action of the liquid substance through the nib may continue slightly. Even when an anti-drip device is part of the applicator device, such slight capillary action may be unavoidable and result in small amounts of liquid substance, such as an acidic liquid, being captured inside the interior cap, at the risk of hitting a user when he/she removes the interior cap to use the applicator device. As a means to prevent these inconveniences, the interior cap may in a preferred embodiment have a proximal blind end part provided with a means for absorbing liquid, in the following also referred to as an "absorbing means", which absorbing means catches, absorbs and traps residual any liquid substances that might be inside the interior cap. So when a user removes the interior cap with the absorbing means, he/she is at no risk of getting the liquid on his/hers hands.

Absorbing means are typically soft and deformable, so to ensure that said absorbing means are installed properly at the blind end of the interior cap, and does not collapse during such insertion, the absorbing means for absorbing a liquid substance may comprise a ring-shaped mount that serves as a cartridge to receive, support and surrounds at least a length of the absorbing means. In this way it is the ring-shaped mount that firmly holds the absorbing means, whereas it is the ring-shaped mount that is force-fitted into engagement with the blind end of the interior cap, to be firmly stucked there, without the absorbing means deforms and looses its absorbing properties. The absorbing means may be glued to the ring-shaped mount to avoid that it drops out unintended of the ring-shaped mount when potentially wetted by liquid substance, or the absorbing means can be fitted into, and attached to, the ring-shaped mount, e.g. simply be engaged by an annular wall of ring-shaped mount against an interior seat of said ring-shaped mount.

The absorbing means may e.g. be a polyester core firmly situated in the ring-shaped mount. Such a core can have the geometrical outline of a short solid cylinder, and e.g. be of a polyester foam, and/or contain super absorbent polymers (SAP). Said ring-shaped mount can be made of any plastic material, e.g. poly propylene, that is able to be create a form-stable structure.

Preferably the ring-shaped mount may have a fluted or corrugated annular wall, which may provide one or both of an inherent shape memory due to some radial flexibility of the annular wall due to the flutings/corrugations; and a large surface area for contact with the absorbing means. The ring-shaped mount may further define and provide a seat for the means for absorbing liquid.

Optionally a fluted or corrugated annular wall may thus confer some inherent flexibility to the ring-shaped mount that contributes to facilitate inserting the ring-shaped mount with the captured absorbing means into the interior cap, and contributes to self-fastening of the ring-shaped mount at the blind end of the interior cap due to inherent shape memory.

In embodiments of applicator devices having child proofing means, said child proofing means prevents children from unauthorized removing any of the exterior proximal cap and the interior cap.

The invention also concerns a method of assembling an applicator device, e.g. in form of a capillary pen, which method comprises the steps of
a) providing a reservoir configured for holding a liquid substance and having a nib-facing proximal end and an opposite distal end,
b) mounting the above-described anti-drip device onto the nib-facing proximal end of the reservoir,
c) inserting the reservoir with the anti-drip device into a holder that is provided with a nib so that the nib extends into an interior bore of the anti-drip device and the nib extends into the reservoir at the nib-facing proximal end of said reservoir to obtain fluid communication between the nib and the liquid substance, to thereby obtain capillary action into the nib of the liquid substance,
d) supplying the liquid substance to the reservoir, and
e) closing the holder using a distal end cap mounted to the distal end of the holder.

The nib may be covered by an interior cover in form of an interior cap to prevent evaporation, volatilizing, spillage, and leakage of the liquid substance during and between uses of the applicator device, such as an applicator pen. A further exterior cover in form of an exterior proximal cap may be applied as well, e.g. as part of a child proofing means. The interior cover and the exterior cover may be an integral cover unit.

The method of assembling an applicator device according to the present invention may thus further comprise step f) of mounting the interior cap to surround the nib, and securing at least said interior cap to the proximal end of the holder. Optionally the exterior proximal cap can be secured around and covering the interior cap.

In some embodiments of methods of assembling applicator devices of the present invention, step f) may advantageously be preceded by step f') that comprises the steps:
- f'1) providing a ring-shaped mount, which preferably has a fluted or corrugated annular wall,
- f'2) providing an absorbing means,
- f'3) mounting the absorbing means into the ring-shaped mount,
- f'4) providing an insertion tool configured to introduce the ring-shaped mount with the absorbing means into the interior cap,
   and
- f'5) using the insertion tool to axially displace the ring-shaped mount with the absorbing means towards a proximal blind end of the interior cap.

A special insertion tool has been developed to insert the ring-shaped mount with the absorbing means at a proximal blind end of the interior cap in a manner so that the absorbing means are not compressed during insertion.

Thus the present invention also relates to an insertion tool for use with the applicator device.

Preferably said special insertion tool is configured complementary to the interior of the interior cap except in being shorter so that axial space is left to move the ring-shaped mount provided with the absorbing means in front of the insertion tool.

To that aspect the insertion tool may e.g. have
- a proximal tubular insertion part with an axially protruding coupling part that fits into a distal opening of the ring-shaped mount, which protruding coupling part has a smaller diameter than the proximal tubular insertion part,
- the proximal tubular insertion part extends axially opposite the protruding coupling part into plural stepped intermediate insertion parts, wherein the steps of the plural stepped intermediate insertion parts have gradually increasing diameters, and
- wherein the largest stepped intermediate insertion part extends into a push plate having a push plate diameter that is larger than the largest diameter of the step of the intermediate insertion parts having the largest diameter.

To insert the assembly of a ring-shaped mount provided with an absorbing means, the tip of the proximal tubular insertion part is inserted into the distal opening of the ring-shaped mount, and the insertion tool is inserted into the interior cap and displaced axially by applying a force to the push plate towards the blind end of the tapering proximal end part of the interior cap.

The axial lengths of the proximal tubular part and of the stepped intermediate insertion parts, respectively, matches the interior of the interior cap, except that the axial length of the proximal tubular part is shorter than the interior depth of the tapering proximal tubular part of the interior cap to allow space for the aforementioned assembly.

The diameter of the push plate is so large that the insertion tool cannot be pressed too far inside the interior cap that the absorbing means gets damaged.

The invention will now be described in further details with reference to the drawing that shows different exemplary embodiments of anti-drip devices of the present invention.
Fig. 1 is an exploded, perspective view, seen from an exterior proximal cap, of an applicator device in form of an exemplary capillary pen implementing a first embodiment of an anti-drip device of the present invention,
Fig. 2 shows the same, but from the opposite end,
Fig. 3 is a perspective side view of the same in assembled state but without exterior proximal cap and interior cap, and without distal end cap,
Fig. 4 is an axial sectional view taken along line IV- IV in Fig. 3, and for illustrative purposes the reservoir is seen without content of liquid substance,
Fig. 5 is an enlarged scale perspective view, seen from the side, of the first embodiment of an anti-drip device, also part of the capillary pen seen in Figs. 1 - 4,
Fig. 6 is an axial sectional view taken along line VI - VI in Fig. 5,
Fig. 7 is a perspective enlarged scale view of a second embodiment of an anti-drip device seen from the proximal end,
Fig. 8 shows the same seen from the side,
Fig. 9 is an axial sectional view taken along line IX - IX in Fig. 7,
Fig. 10 is a perspective enlarged scale view of a third embodiment of an anti-drip device seen from the proximal end,
Fig. 11 is an axial sectional view taken along line XI -XI in Fig. 10,
Fig. 12 is a perspective enlarged scale view of a fourth embodiment of an anti-drip device seen in an inclined side view,
Fig. 13 is an axial sectional view taken along line XIII -XIII in Fig. 12,
Fig. 14 is a perspective enlarged scale view of a fifth embodiment of an anti-drip device seen from the proximal end,
Fig. 15 is an axial sectional view taken along line XV -XV in Fig. 14,
Fig. 16 is a perspective enlarged scale side view of a sixth embodiment of an anti-drip device seen from the proximal end,
Fig. 17 is an axial sectional view taken along line XVII -XVII in Fig. 16,
Fig. 18 is an exploded perspective side view of a modified applicator device,
Fig. 19 shows, in perspective, the same seen from the distal end of the modified applicator device,
Fig. 20 shows, in an enlarged scale, a perspective view of the interlocking disc seen from the side facing towards the interior cap,
Fig. 21 shows, in perspective, the same seen from the opposite side,
Fig. 22 shows, in an enlarged scale, a perspective view of the interior cap seen from the free proximal end part,
Fig. 23 shows a perspective view of the interior cap seen from its distal end part,
Fig. 24 is an enlarged sectional view taken along the lengthwise extending axis of the exterior proximal cap seen in figs. 18 and 19,
Fig. 25 is a perspective side view of the ring-shaped mount, seen in an enlarged scale,
Fig. 26 shows the same seen from its distal end,
Fig. 27 is a perspective view of an absorbing means, seen in an enlarged scale,
Fig. 28 shows, in an enlarged scale perspective view, an assembly of an absorbing means and a ring-shaped mount seen from the absorbing means,
Fig. 29 shows the same seen from the opposite end,
Fig. 30 shows the same seen from the side,
Fig. 31 shows the applicator device of figs. 18 and 19 in assembled stated, and in sectional view taken along the lengthwise extending axis,
Fig. 32 is a sectional view taken along the lengthwise extending axis of an applicator device without child proofing means,
Fig. 33 is a perspective view of an insertion tool designed to insert the ring-shaped mount with the absorbing means into the interior cap, and

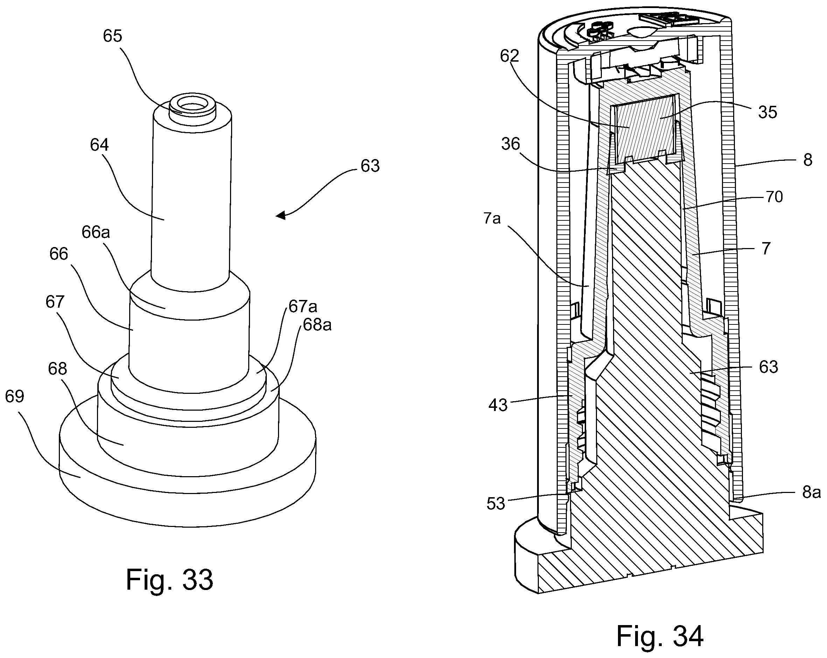

Fig. 33 is an axial sectional view of the interior cap inside the exterior proximal cap, where the ring-shaped mount with the absorbing means into the interior cap is seen inserted into the interior cap by means of the insertion tool.

The dimensions and shapes of the components of the various embodiments of applicator devices seen in the figures are examples, and other shapes and mutual dimensions are within the scope of the present invention.

The substance (not shown in the figures) hold by the reservoir can be any kind of substance, but is preferably a liquid. The applicator device can be any kind of applicator implementing a reservoir for local administration of a substance. Besides medical application, the koncepts of the anti-drip device of the present invention and of the the interior cap with an assemply of an absorbing mean of mounted in a ring-shaped mount can e.g. be used in an ink or correction fluid applicator pen, perfume applicator pens, and deodorants.

In the exemplary embodiments discussed below the applicator device is described as a capillary pen for treating warts or fungus. The tubular reservoir holds a liquid substance, such as formic acid. According to the Chemical Abstracts Service formic acid has a density at 20°C of 1.220 g/mL, and an acidic pH, thus is chemically harsh, and should not be put on skin locations not to be treated. It is therefore highly important to avoid splashing and dripping from said capillary pen.

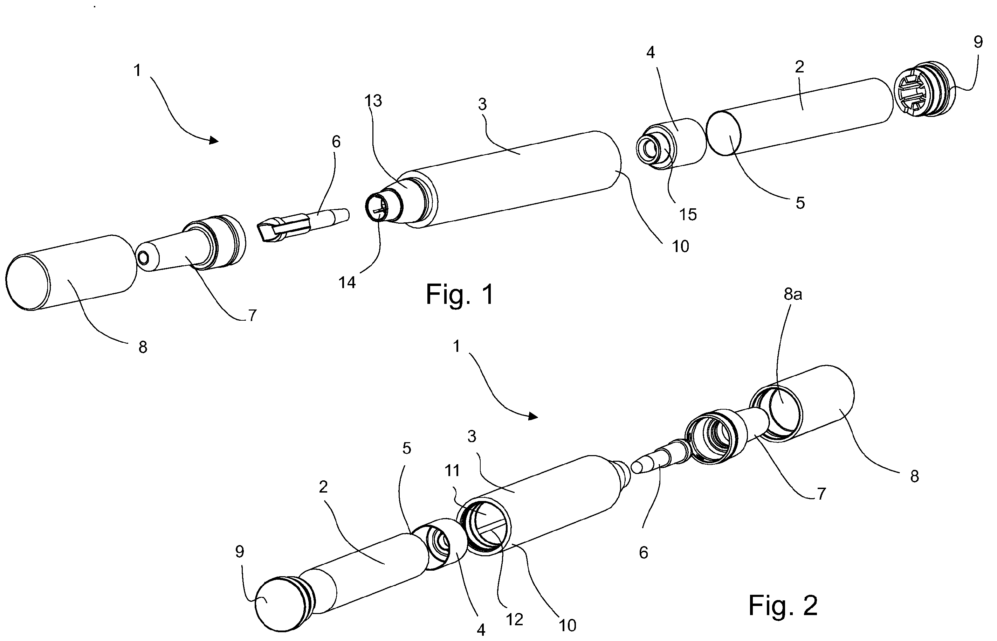

Figs. 1 - 4 show a capillary pen 1 comprising the following main components: A reservoir 2 in form of a schematically shown reservoir holding a liquid, e.g. formic acid, a holder 3 to receive and accommodate the reservoir 2, an anti-drip device 4 to be mounted to a nib-facing proximal end 5 of the reservoir 2 and to capture dripping liquid, a nib 6, an interior cap 7 for surrounding the nib 6, an exterior proximal cap 8 that optionally may be made integral with the interior cap 7 and enclose said interior cap, and a distal cap 9 to end-close a distal end 10 of the holder 3.

The interior wall 11 of the holder 3 may have axially and radially extending grooves 12, of which only a few is seen in the view of Fig. 2, to provide a gap to the reservoir 2.

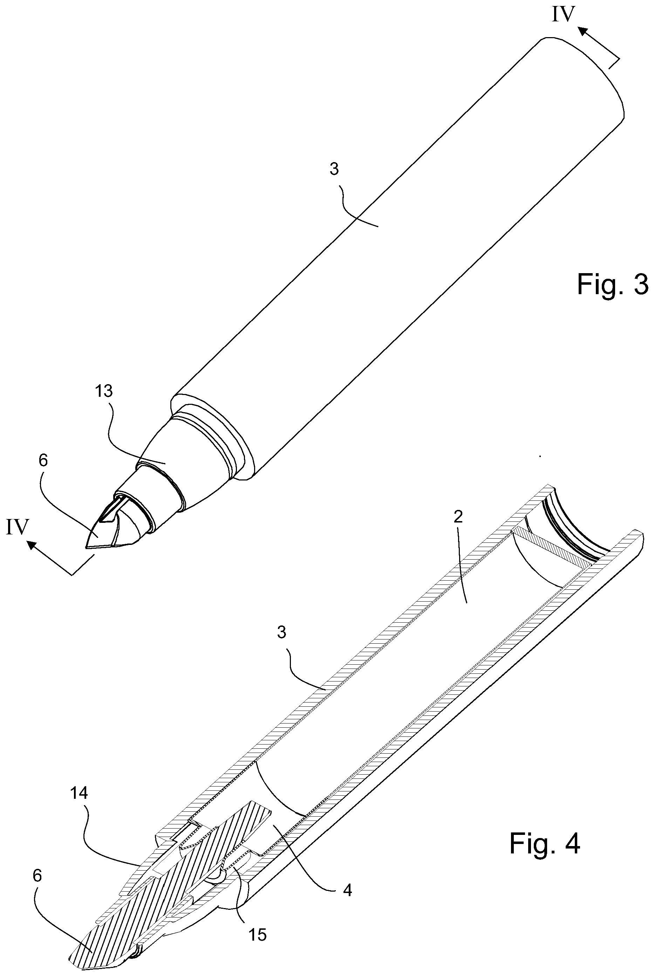

A proximal end 13 of the holder 3 tapers stepwise towards its proximal free end, and has an interior seat or constrictions 14 for receiving at least a part of a proximal nib-facing end part 15 of the anti-drip device 4, as seen best in Figs. 2 and 4.

As seen in Fig. 3 the nib 6 protrudes from the proximal end 13 of the holder 3. Said nib 6 serves as a wick. The nib 6 is shown as a solid body in the sectional view of Fig. 4, but might be porous, and/or have capillary channels to promote liquid uptake and flow through it. The nib 6 may e.g. be made of a material like compressed porous felt or sponge, thus of materials that posses capillary properties, and do not deform when manipulated, thus is able to keep their 3D nib shape.

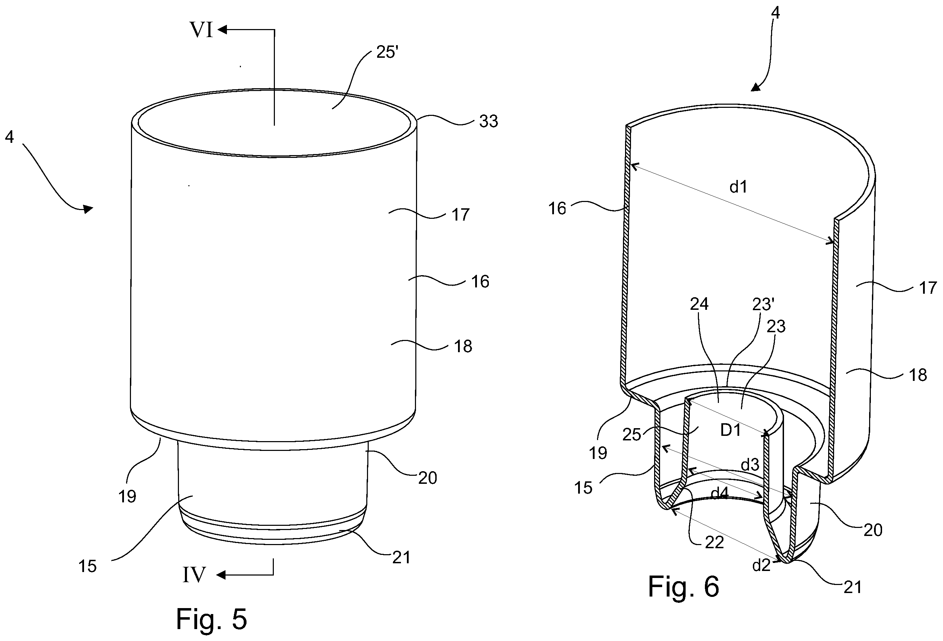

The first embodiment of an anti-drip device 4, which is implemented in the capillary pen 1 seen in Figs. 1 - 4, is seen in enlarged scale views in Figs. 5 and 6.

As shown in Figs. 5 and 6 the first embodiment of an anti-drip device 4 according to the present invention resembles an inverted sleeve.

An exterior tubular anti-drip part 16 is delimited by an exterior annular wall 17. The exterior annular wall 17 has an exterior distal annular wall part 18 that has a first interior diameter d1, which exterior distal annular wall part 18, via a shoulder 19, extends into an exterior proximal annular wall part 20 having a third interior diameter d3 that is smaller than the first interior diameter d1.

The exterior proximal annular wall part 20 extends via a U-bend wall part 21 into the wall 22 of an interior anti-drip part 23, whereby a proximal opening with a second interior diameter d2 that is smaller than the first interior is delimited. An interior annular wall part 24 of the interior anti-drip part 23 has an exterior diameter D1 that is equal to or smaller than the third interior diameter d3 and a fourth interior diameter d4 that fits around the nib 6, which fourth interior diameter are also a diameter of an interior bore 25 of the interior anti-drip part 23.

The U-bend wall part 21 provides a proximal free funnel-shaped nib-facing end part 15 of the anti-drip device 4 that facilitates insertion of the nib 6 into an interior bore 25 delimited by the wall 22 of the interior anti-drip part 23. The exterior proximal annular wall part 20 delimits an exterior bore 25' of the anti-drip device 4.

It may be preferred for some embodiments of the first embodiment of an anti-drip device 4 that an interior distal free end 23' of the interior tubular anti-drip part 23 protrudes inside the exterior tubular anti-drip part 16 beyond the shoulder 19, thus longer inside the exterior tubular anti-drip part 16 than the level of the shoulder 19, whereby the interior distal free end of the interior tubular anti-drip part 23 is closer to a distal annular edge 33 of the anti-drip device 4 than the shoulder 19.

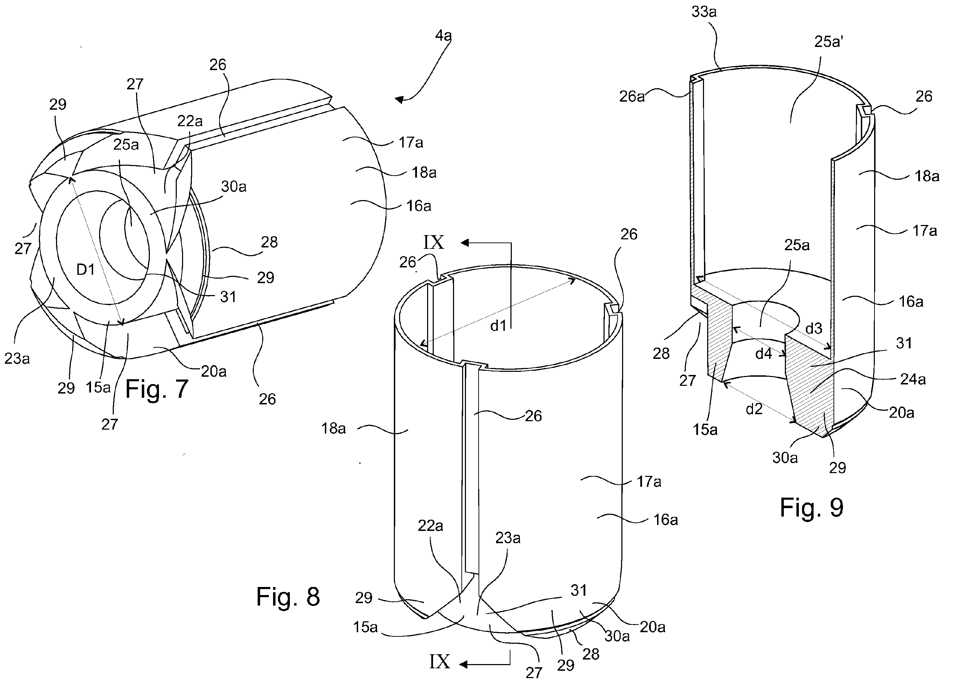

Figs. 7, 8 and 9 show a second embodiment of an anti-drip device 4a according to the present invention. The second embodiment of an anti-drip device 4a functions in the same manner as the first embodiment of an anti-drip device 4 and has similar components. For functional similar parts same reference numerals are used except that the annotation "a" has been added to said reference numerals.

An exterior tubular anti-drip part 16a is delimited by an exterior annular wall 17a that has three axially extending grooves 26 equidistantly spaced apart to receive corresponding ribs 12 of the holder 3. The exterior annular wall 17a has an exterior distal annular wall part 18a that extends directly into an exterior proximal annular wall part 20a that partly surrounds an interior anti-drip part 23a due to being shorter at the three axially extending grooves 26. At each axially extending groove 26 the exterior proximal annular wall part 20a has a V-shaped cut-out 27 arranged with its apex towards a proximal end of a respective groove 26. Thereby a proximal edge 28 of the exterior proximal annular wall part 20a has alternating female features, the V-shaped cut-outs 27, and male features, the remaining longer parts 29 of said exterior proximal annular wall part 20a, respectively, whereby the proximal end 15a of the second embodiment of the anti-drip device 4a gets a wavy coupling end 30 that can be shaped at least partly complementarily to the interior shape of the nib-facing end 13 of the holder 3. The cut-outs 27 are in the present embodiment of an anti-drip device 4a V-shaped, but other shapes are within the scope of the present invention, such as U-shapes and right-angled.

Emphasis is made that the holder 3 can have more or less, including none, than three axially extending ribs 12, and they do not need to be spaced equidistantly if present. The number and arrangement of grooves 26 should preferably be complementary to that of the ribs 12. However a rib 12 may be radially longer than the depth of a groove 26 so that a venting gap remains between the holder 3 and the reservoir 2.

The interior anti-drip part 23a is a ring-shaped body 31 made integral with the exterior proximal annular wall part 20a of the exterior annular wall 17a at the proximal end 15a of the anti-drip device 4a. An interior bore 25a is delimited by the wall 22a of the interior anti-drip part 23a.

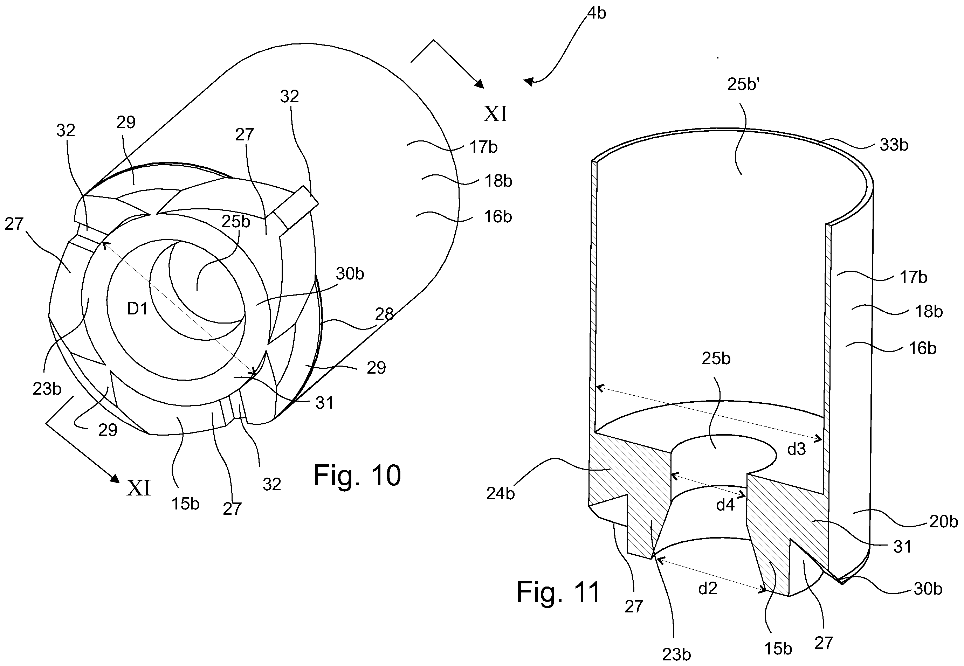

Figs. 10 and 11 show a third embodiment of an anti-drip device 4b according to the present invention. The third embodiment of an anti-drip device 4b corresponds to the second embodiment of an anti-drip device 4a, and it functions in the same manner as the second embodiment of an anti-drip device 4a, and have similar components. For functional similar parts same reference numerals are used except that the annotation "b" has been added to said reference numerals instead of the annotation "a".

The third embodiment of an anti-drip device 4b differs from the second embodiment of an anti-drip device 4a only in a modified proximal end 15b, and in that it does not have grooves.

The V-shaped cut-outs 27 extend axially a short distance into the exterior distal annular wall part 18b to thereby provide associated axial recesses 32 to assist in locating the anti-drip device 4b properly into the interior seat 14 at the proximal end 13 of the holder 3, which interior seat 14 may be configured to mate the recesses 32.

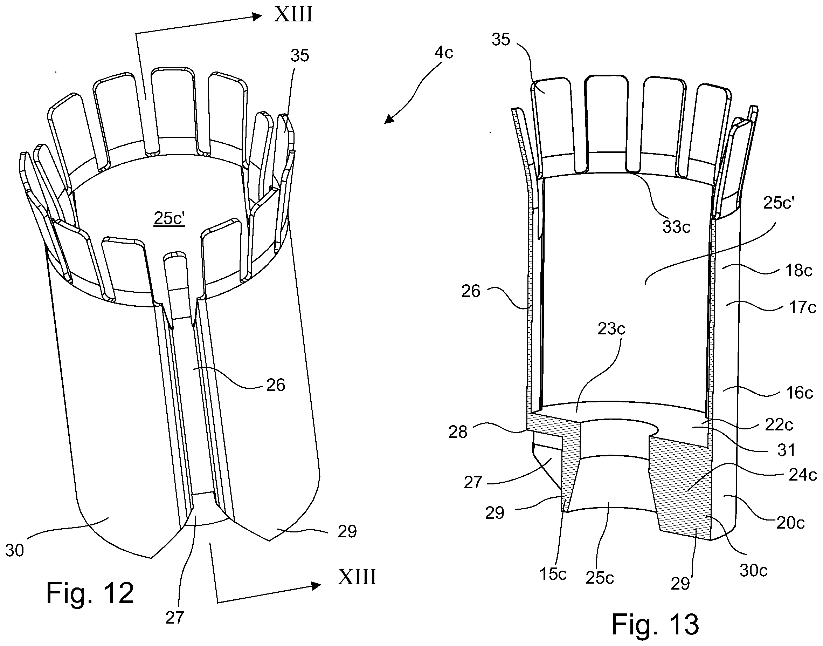

Figs. 12 and 13 show a fourth embodiment of an anti-drip device 4c according to the present invention. The fourth embodiment of an anti-drip device 4c corresponds to the second embodiment of an anti-drip device 4a. For functional similar parts same reference numerals are used, except that the annotation **"c"** replaces the annotation "a".

The only difference between the fourth embodiment of an anti-drip device 4c and the second embodiment of an anti-drip device 4a is that a distal annular edge 33 of the exterior tubular anti-drip part 16a has a plurality of spaced apart flexible protruding fingers 35 to avoid deformation of the reservoir 2 when mounting the anti-drip device 4c.

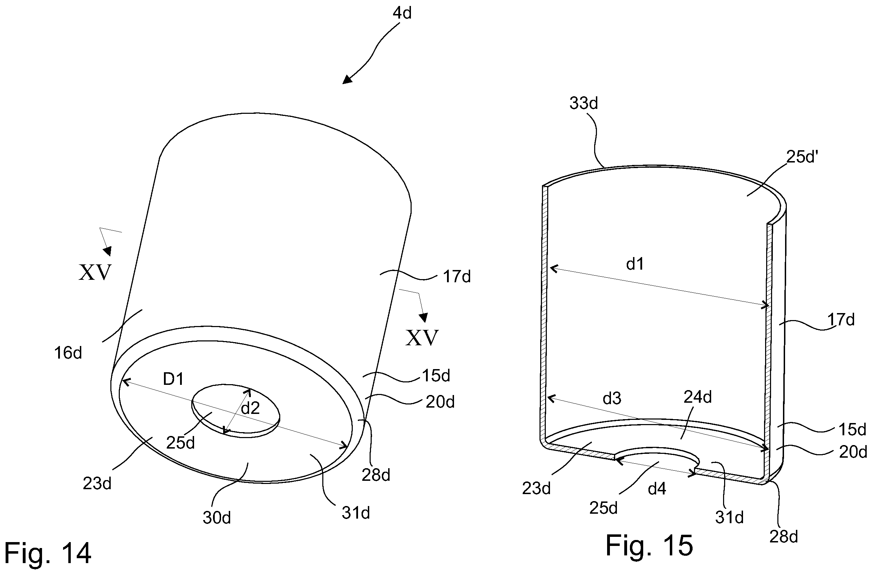

Figs. 14 and 15 show a simple fifth embodiment of an anti-drip device 4d that resembles the previous described embodiments of anti-drip devices. For like parts same reference numerals are used, except for the annotation "d" being used.

The fifth embodiment of an anti-drip device 4d has an interior anti-drip part 23d in form of a ring-shaped body 31d made integral with an exterior proximal annular wall part 20d of the exterior annular wall 17d at the proximal end 15d of the anti-drip device 4d. Thus at the proximal edge 28d of the exterior proximal annular wall part 20d, said exterior proximal annular wall part 20d extends at substantially right angle into the ring-shaped body 31d. An interior bore 25d is provided in the substantially disc-shaped thin ring-shaped body 31d to receive the nib 6. The fifth embodiment of an anti-drip device 4d is both simple and inexpensive to manufacture, and to mount at the nib-facing proximal end 5 of the reservoir 2.

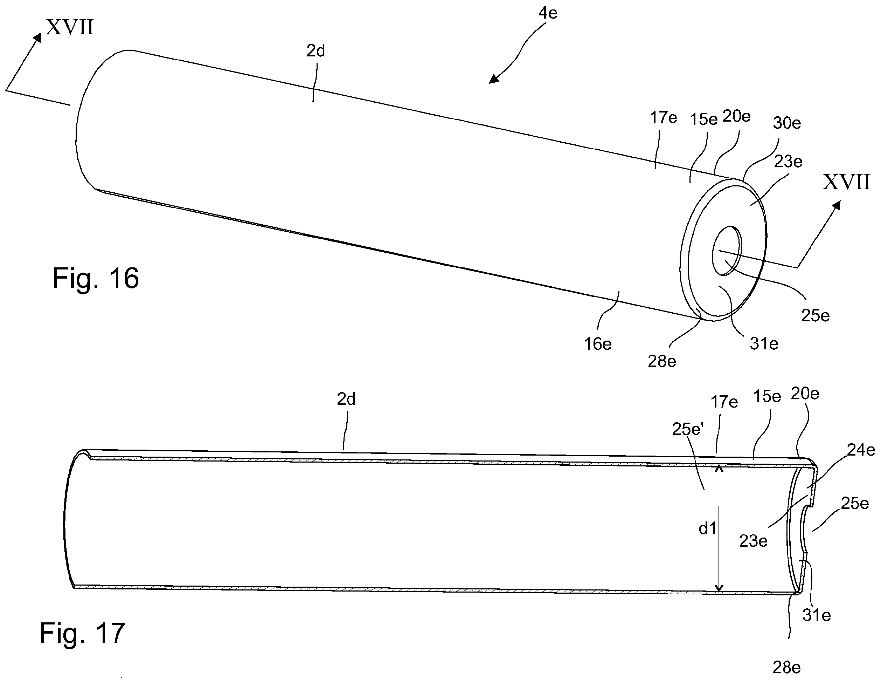

Figs. 16 and 17 show a sixth embodiment of an anti-drip device 4e, which is a modification of the fifth embodiment of an anti-drip device 4d. For like parts same reference numerals are used except that the annotation "e" is used instead of the annotation "d".

The sixth embodiment of an anti-drip device 4e differs from the fifth embodiment of an anti-drip device 4d in that it has an elongated exterior proximal annular wall part 20e with a length sufficient to constitute the reservoir 2d to be filled with porous material like cotton or a sponge material, which subsequently is soaked with liquid substance.

Any of the anti-drip devices 4;4a;4b;4c;4d;4e can be an integral part of the holder 3, thus be co-molded with the holder 3, instead of being a separate part. The reservoir 2 is then inserted by a simple axial displacement into the holder 3. In doing so the end cap 9 may be the part that eventually forces the axial displacement of the reservoir 2 fully into the anti-drip device 4;4a;4b;4c;4d;4e so that the nib 6 gets into capillary communication with the substance inside the reservoir 2.

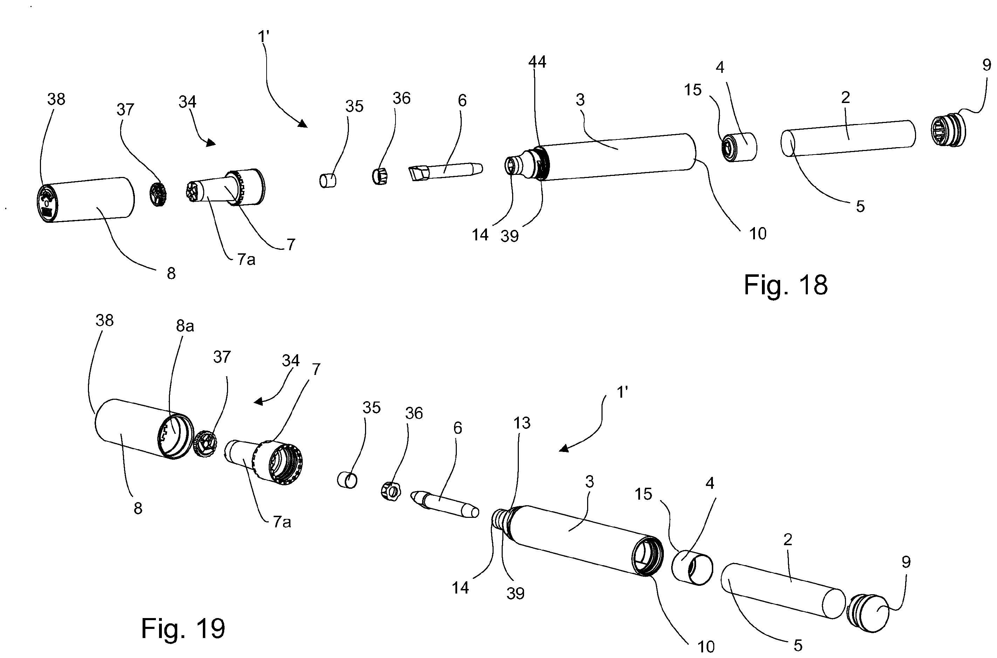

Fig. 18 is an exploded perspective side view of a modified applicator device 1'. The modified applicator device 1' resembles the applicator device 1 described in relation to the previous figures, and for like parts same reference numerals are used.

The modified applicator device 1' differs in that it has a child proofing means 34; and in that the interior cap 7 is provided with an absorbing means 35 fixed in an annular holder 36 in form of a ring-shaped mount 36.

The child proofing means 34 is based on known principles of "press open child proofing" resembling the dual action safety mechanisms on products, like medicine bottles, bottles with sanitary solutions, acids, bases, etc., that require an adult's specific action-pressing and twisting a button while simultaneously opening to defeat a lock, thereby preventing young children from accessing dangerous contents or areas like medicines and chemicals. The dual-action safety mechanism combines two movements: pushing of a part and rotating of a/the part.

In respect of the modified applicator device 1', the exterior proximal cap 8 is the part that is pushed towards the distal cap 9 and used as an actuator to rotate the interior cap 7 off the holder 3.

Subject to a sufficient axial pushing force the exterior proximal cap 8 is thus used as the pushing means that puts an interlocking disc 37, which is arranged, e.g. snap fitted or secured by gluing, inside a proximal end 38 of the exterior proximal cap 8, into interlocking engagement with an exterior free proximal end part 39 of the interior cap 7.

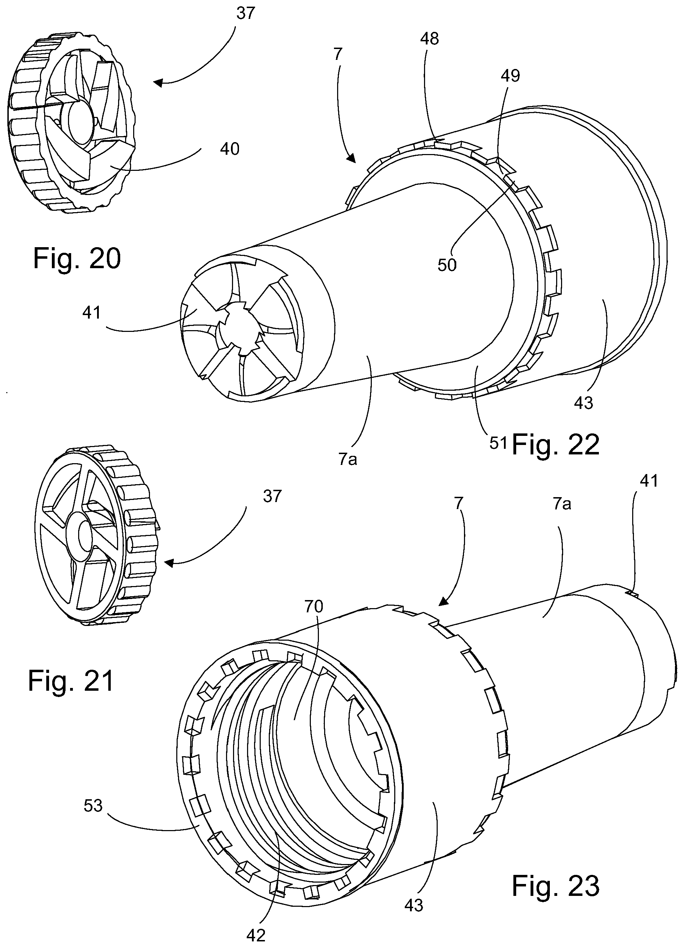

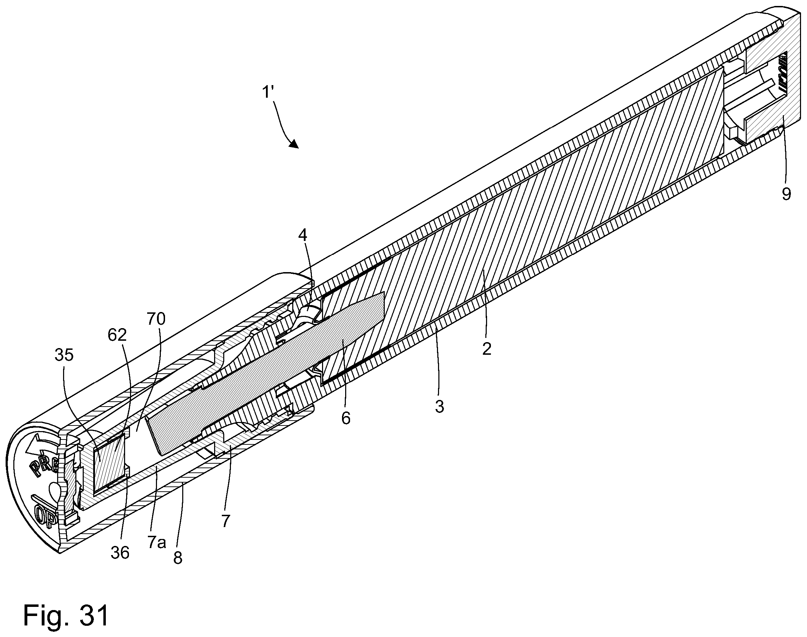

The interlocking disc 37 may have first engagement means 40, such as male or female engagement means, as seen best in the enlarged scale views of figs. 20 and 21, configured to interlock with complementary shaped second engagement means 41, such as female or male engagement means, at the exterior free proximal end part 7a of the interior cap 7, as seen best in the enlarged scale views of figs. 22 and 23, and in fig. 31. The first engagement means 40 and the complementary shaped second engagement means 41 can e.g. be configured as notches, protrusions, indents, or ratchets that only engage when an axial distance between the interlocking disc 37 and the exterior free proximal end part 7a is eliminated when pushing the exterior proximal cap 8 axially towards the interior cap 7.

Once secure engagement between the first engagement means 40 and the complementary shaped second engagement means 41 has been achieved, rotation of the exterior proximal cap 8 about its longitudinal axis is directly transferred to a rotation of the interior cap 7, and as the axial displacement of the exterior proximal cap 8 has also moved the interior cap 7 closer to the proximal end 13 of the holder 3, an interior threading 42 of a distal end part 43 of the interior cap 7 has been made to engage with an exterior threading 44 at the proximal end 13 of the holder 3 to allow unscrewing of the interior cap 7 from the holder 3, - thus by rotating the exterior proximal cap 8 to bring the interior cap 7 along.

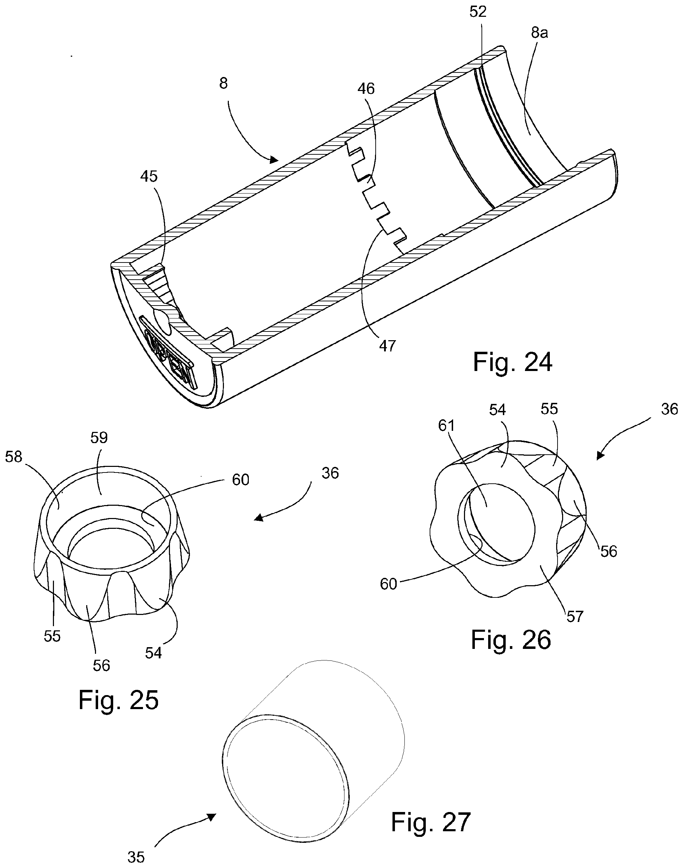

As seen in fig. 24, which is a sectional view along the lengthwise extending axis of the exterior proximal cap 8 seen in figs. 18 and 19, said embodiment of the exterior proximal cap 8 has an interior seat 45, for fastening the interlocking disc 37 in an anti-rotational manner.

Said exterior proximal cap 8 further has third engagement means 46 in form of an interior ring of first teeth 47 that is arranged protruding radially inside the exterior proximal cap 8 to engage a fourth engagement means 48 in form of an exterior ring of gaps or indents 49 between second teeth 50 at a proximal end 51 of the distal end part 43 of the interior cap 7, to thereby further ensure that the interior cap 7 can co-rotate with the exterior proximal cap 8 to be screwn off the holder 3, thus bypassing the child proofing means 34 as described above. Thus the third engagement means 46 and the fourth engagement means 48 are extra means to ensure that the exterior proximal cap 8 can push and rotate the interior cap 7 to bypass the deliberate initial distance between the interior threading 42 and the exterior threading 44 that previously prevented said threadings 42,44 from engaging, to unscrew the interior cap 7.

As further seen in fig. 24 the exterior proximal cap 8 has an annular bead 52 to snap below a free end 53 of the distal end part 43 of the interior cap 7 to ensure that the exterior proximal cap 8 does not unintentionally drop off, but on the other hand easily can be used to unscrew the interior cap 7.

Figs. 25 and 26 shows that a ring-shaped mount 36 is configured as a frustum of a cone having an annular wall 54, which has a plurality of spaced apart flutes 55 or similar indents, alternating with protrusions 56, whereby a distal end 57 of said ring-shaped mount 36 gets a wawy perimeter, as seen in fig. 26. The annular wall 54 delimits a bore 58 to receive the absorbing means 36 seen in fig. 27. The absorbing means 37 is inserted through a proximal opening 59 of the bore 58, thus at the top of the frustum of a cone, as seen in the orientation of fig. 25, to abut and rest on a seat 60 at a distal opening 61 of the bore, thus at the bottom of the frustum of a cone, as seen in the orientation of fig. 25. This distal opening 61 is smaller than the proximal opening 59 to prevent the absorbing means 36 from passing through.

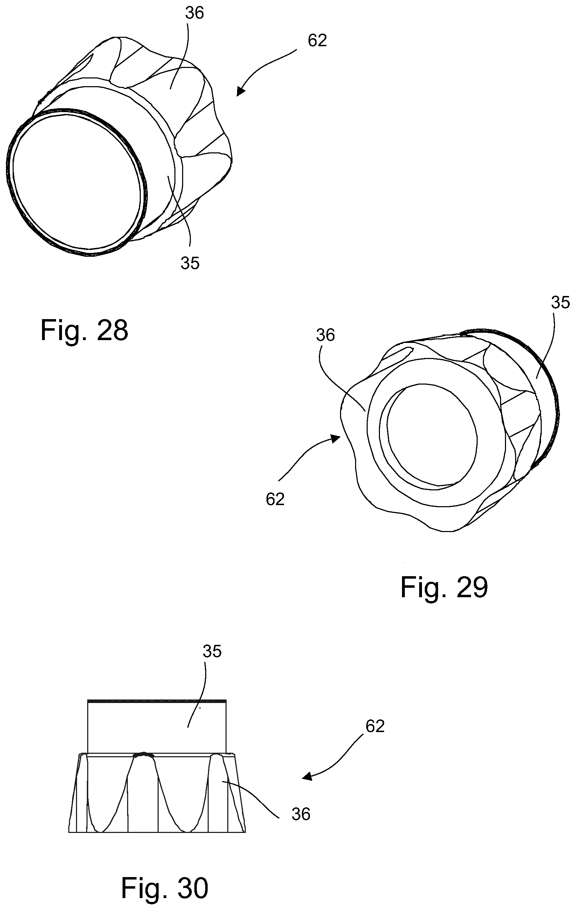

Figs. 28, 29 and 30 show an assembly 62 of an absorbing means 35 and a ring-shaped mount 35 in various views ready for being inserted into the interior cap 7. The absorbing means protrudes axially from the ring-shaped mount 36 at the proximal opening 59 of the bore 58 of the ring-shaped mount 36.

Fig. 31 shows the applicator device 1' in assembled stated and in sectional view taken along its lengthwise extending axis and illustrates the position of the assembly 62 of an absorbing means 35 and a ring-shaped mount 35 inserted into the tapering exterior free proximal end part 7a of the interior cap 7, thus fitted firmly to be ready to be situated at the interior blind end of said tapering exterior free proximal end part 7a.

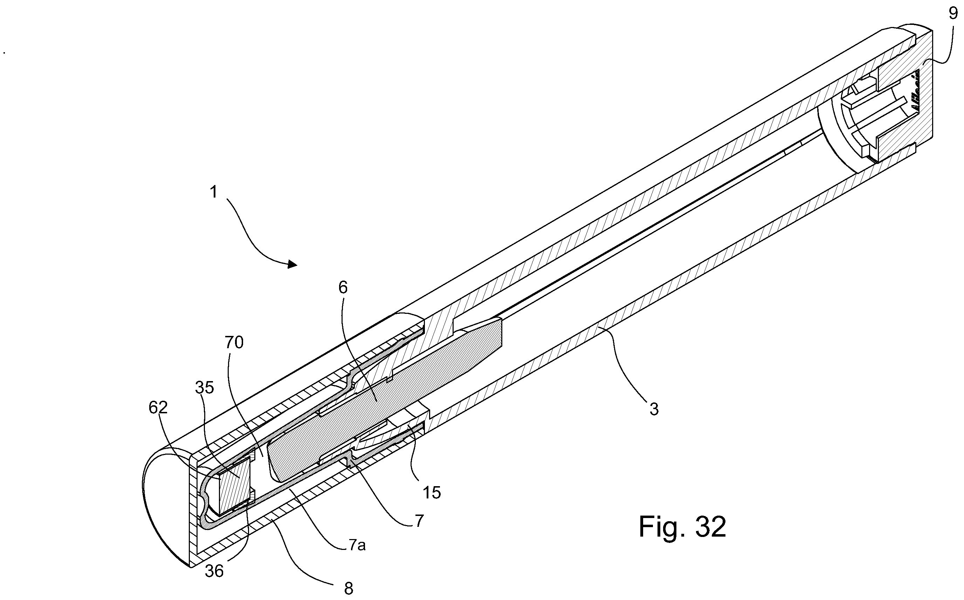

Fig. 32 is a sectional view taken along the lengthwise extending axis of the applicator device 1 discussed in relation to figs. 1 and 2, thus without the child proofing means, but incorporating the assembly 62 of an absorbing means 35 and a ring-shaped mount 35 inserted into the tapering exterior free proximal end part 7a of the interior cap 7. The reservoir 2 has not yet been inserted into the holder 3.

Fig. 33 is a perspective view of an insertion tool 63 that has an exterior shape, and exterior dimensions, selected to prevent that the interior cap 7 is pushed too far inside the interior cap 7.

Fig. 34 is an axial sectional view of the interior cap 7 inside the exterior proximal cap 8, where the ring-shaped mount 36 with the absorbing means 35 has been inserted into the interior cap 7 by means of the insertion tool 63 seen in fig. 33.

The insertion tool 63 has a proximal tubular insertion part 64 with an axially protruding coupling part 65 that fits into the distal opening 61 of the ring-shaped mount 36. The protruding coupling part 65 has a smaller diameter than the proximal tubular part 64 so that the ring-shaped mount 36 can be situated on a free tip 65 of the proximal tubular insertion part 64.

The proximal tubular insertion part 64 extends axially opposite the protruding coupling part 65 into plural stepped intermediate insertion parts 66,67,68, before the insertion tool 63 is terminated by a push plate 69, to which an axial force is applied during insertion of the insertion tool equipped with the assembly 62 into the lumen 70 of the interior cap 7, as seen best in fig. 34.

A first intermediate insertion part 66 has a first intermediate diameter that is larger than the diameter of the proximal tubular insertion part 64 whereby a first annular shoulder 66a is obtained, which annular shoulder 66a cannot pass the constriction between the exterior free proximal end part 7a of the interior cap 7 and the distal end part 43 of the interior cap 7.

The first intermediate insertion part 66 extends into a second intermediate insertion part 67 that has a second intermediate diameter that is larger than the first intermediate diameter 64 whereby a second annular shoulder 67a is obtained, which second annular shoulder 67a is sized not to pass below a free end 53 of the distal end part 43 of the interior cap 7.

The second intermediate insertion part 67 extends into a third intermediate insertion part 68 having a third diameter that is larger than the second diameter and provides a third shoulder 68a so that the diameter of third intermediate insertion part 68 is substantially the same as the interior diameter of the exterior proximal cap 8 at its opening 8a.

The third intermediate insertion part 68 extends into push plate 69 that has a push plate diameter that is sufficiently larger than the third diameter so that the insertion tool 63 cannot be pushed too far up inside the lumen 70 of the interior cap 7.

The features and components of the various embodiments of anti-drip devices may be combined. E.g. can any of the embodiments of anti-drip devices be provided with protruding fingers. The second, third, fourth, fifth and sixth embodiment of anti-drip devices can have an interior anti-drip part that protrudes longer upright into the interior anti-drip part, but shorter interior anti-drip parts are also within the scope of the present invention.

## Claims

1. An anti-drip device (4;4a;4b;4c;4d;4e) configured for mounting to an applicator device (1;1') of the kind comprising a reservoir (2;2d) holding a substance, and a nib (6) in fluid communication with the substance inside the reservoir (2;2d), **characterised in that** the anti-drip device (4;4a,4b;4c;4d;4e) comprises
- an exterior tubular anti-drip part (16;16a;16b;16c;16d;16e) configured for being situated around the reservoir (2;2d), and
- an interior anti-drip part (23;23a;23b;23c;23d;23e), at least a part of which is arranged inside the exterior tubular anti-drip part (16;16a;16b;16c;16d;16e), and having an interior bore (25; 25a; 25b; 25c; 23d; 23e) configured for sealingly receiving the nib (6).

2. An anti-drip device (4;4a;4b;4c;4d;4e) according to claim 1, **characterised in that**
- an exterior annular wall (17;17a;17b;17c;17d;17e) of the exterior tubular anti-drip part (16;16a;16b;16c;16d;16e) comprises an exterior distal annular wall part (18;18a;18b;18c;18d;18e) having a first interior diameter (d1).

3. An anti-drip device (4;4a;4b;4c;4d;4e) according to any of the preceding claims 1 or 2, **characterised in that**
- at least a part of an interior annular wall part (24;24a;24b;24c;24d;24e) of the interior tubular anti-drip part (23;23a;23b;23c;23d;23e) has a second interior diameter (d2) that is smaller than the first interior diameter (d1).

4. An anti-drip device (4;4a,4b;4c;4d;4e) according to any of the preceding claims, **characterised in that**
- the exterior distal annular wall part (18;18a;18b;18c;18d;18e) of the exterior tubular anti-drip part (16;16a;16b;16c;16d;16e) extends into an exterior proximal annular wall part (20;20a;20b;20c;20d;20e) of the exterior tubular anti-drip part (16;16a;16b;16c;16d;16e), at least a part of said exterior proximal annular wall part (20;20a;20b;20c;20d;20e) has a third interior diameter (d3) that is smaller than or equal to the first interior diameter (d1), and
- at least an interior annular wall part (24;24a,24b;24c;24d;24e) of the interior tubular anti-drip part (23;23a;23b;23c;23d;23e) has an exterior diameter (D1) that is equal to or smaller than the second interior diameter (d2), and the bore has a fourth interior diameter (d4) that is smaller than or equal to the second interior diameter (d2).

5. An anti-drip device (4;4a;4b;4c;4d;4e) according to any of the preceding claims, **characterised in that** the interior tubular anti-drip part (23;23a;23b;23c;23d;23e) is a ring-shaped body manufactured integral with the exterior annular wall (16;16a;16b;16c;16d;15e) and delimiting the interior bore (25;25a;25b;25c;25d;25e) for receiving the nib (6).

6. An anti-drip device (4;4a;4b;4c;4d;4e) according to any of the preceding claims, **characterised in that** the exterior tubular anti-drip part (16;16a;16b;16c;16d;16e) and the interior tubular anti-drip part (23;23a;23b;23c;23d;23e) are arranged so that their respective exterior bore (25';25a';25b';25c';25d';25e') and interior bore (25;25a;25b;25c;25d;25e) are coaxial.

7. A anti-drip device (4;4a;4b;4c;4d;4e) according to any of the preceding claims, **characterised in** comprising one or more of
- the proximal end part (15;15a;15b;15c;15d;15e) of the anti-drip device (4a;4b;4c) has a coupling end (30;30a;30b;30c;30d;30e) shaped complementary to an interior seat (14) of a holder (3), and
- a distal annular edge (33;33a;33b;33c;33d;33e) of the exterior tubular anti-drip part (16;16a;16b;16c;16d;16e) has a plurality of spaced apart flexible protruding fingers (35).

8. An anti-drip device (4a) according to any of the preceding claims, **characterised in that** the exterior tubular anti-drip part (16a) has at least one axially extending groove (26) extending along the axial length of the exterior annular wall (17).

9. An applicator device (1;1') in form of a capillary applicator pen comprising the anti-drip device (4;4a,4b;4c;4d;4e) according to any of the preceding claims 1 - 8, and a reservoir (2;2d) holding a liquid substance.

10. An applicator device (1;1') according to claim 9, **characterised in that** the liquid substance is selected for use in the treatment of dermatological disorders, including treatment of warts and fungus.

11. An applicator device (1;1') according to any of claims 9 or 10, **characterised in** further comprising an interior cap (7) adapted for surrounding and covering at least a free end of the nib (6).

12. An applicator device (1;1') according to claim 11, **characterised in** further comprising an exterior proximal cap (8) configured to enclose said interior cap (7).

13. An applicator device (1;1') according to any of the preceding claims 9 - 12, **characterised in** further comprising a distal cap (9) adapted to end-close a distal end (10) of a holder (3) for a reservoir (2) for the liquid substance.

14. An applicator device (1;1') according to any of claims 12 or 13, **characterised in that** the interior cap (7) and the exterior proximal cap (8) are integral with each other or detachable from each other.

15. An applicator device (1;1') according to any of the preceding claims 11 - 14, **characterised in that** the interior cap (7) has a proximal blind end part (7a) provided with an absorbing means (35) for absorbing liquid.

16. An applicator device (1;1') according to claim 15, **characterised in** comprising a ring-shaped mount (36) to accommodate the absorbing means (35).

17. An applicator device (1;1') according to claim 16, **characterised in that** the ring-shaped mount (36) has a flutted or corrugated annular wall (54) and defines a seat (60) for the absorbing means (35).

18. An applicator device (1;1') according to any of the precedings claims 9 - 17, **characterised in** comprising a child proofing means (34).

19. A method of assembling an applicator device (1;1'), e.g. in form of a capillary pen, which method comprises the steps of
a) providing a reservoir (2) configured for holding a liquid substance and having a nib-facing proximal end (5) and an opposite distal end,
b) mounting the anti-drip device (4;4a,4b;4c;4d;4e) according to any of the preceding claims 1 - 8 onto the nib-facing proximal end (5) of the reservoir (2),
c) inserting the reservoir (2) with the anti-drip device (4;4a,4b;4c;4d;4e) into a holder (3) that is provided with a nib (6) so that the nib (6) extends into an interior bore (25;25a,25b;25c;25d;25e) of the anti-drip device (4;4a,4b;4c;4d;4e) and the nib (6) extends into the reservoir (2) at the nib-facing proximal end (5) of said reservoir (2) to obtain fluid communication between the nib (6) and the liquid substance, to thereby obtain capillary action into the nib (6) of the liquid substance,
d) suppplying the liquid substance to the reservoir (2), and
e) closing the holder (3) using a distal end cap (9) mounted to the distal end (10) of the holder (3).

20. A method of assembling an applicator device (1;1') according to claim 19, **characterised in** further comprising step f) of mounting an interior cap (7) to surround the nib (6), and securing at least the interior cap (7) to the proximal end (13) of the holder (3), optionally securing an exterior proximal cap (8) around the interior cap (7).

21. A method of assembling an applicator device (1;1') according to claim 20, **characterised in that** step f) is preceded by step f') comprising the steps of
- f'1) of providing a ring-shaped mount (36), which preferably had a flutted annular wall (54),
- f'2) of providing an absorbing means (35) in form of a means for absorbing a liquid substance,
- f'3) of mounting the absorbing means (35) into the ring-shaped mount (36),
- f'4) of providing an insertion tool (63) to introduce the ring-shaped mount (36) with the absorbing means (35) into a lumen (70) of the interior cap (7),
- and
- f'5) of using the insertion tool (63) to insert the ring-shaped mount (36) with the absorbing means (35) at a proximal blind end of the interior cap (7).

22. An insertion tool (63) configured to insert a ring-shaped mount (36) with an absorbing means (35) at a proximal blind end of an interior cap (7) of an applicator device (1;1') according to any of the preceding claims 9 - 18 by using the method according to any of the preceding claims 19 - 21, **characterised in that** the insertion tool (63) has
- a proximal tubular insertion part (64) with an axially protruding coupling part (65) that fits into a distal opening (8a) of the ring-shaped mount (36), which protruding coupling part (65) has a smaller diameter than the proximal tubular insertion part (64),
- the proximal tubular insertion part (64) extends axially opposite the protruding coupling part (65) into plural stepped intermediate insertion parts (66,67,68), wherein the steps of the plural stepped intermediate isertion parts (66,67,68) have gradually increasing diameters, and
- wherein the largest intermediate isertion part (66,67,68) extends into a push plate (69) having a push plate diameter that is larger than the diameter the largest intermediate insertion part (66,67,68).
